# EUROPEAN PATENT SPECIFICATION

(11) **EP 3 645 093 B1**
(45) Date of publication and mention of the grant of the patent: **12.10.2022**
(21) Application number: 17804920.1
(22) Date of filing: 30.11.2017
(51) Int. Cl.: A61M 16/00, A61M 16/20, A61M 16/08, A61M 16/10, A61M 16/12

(54) **RESUSCITATION BAG WITH DERIVATION CONDUCT COMPATIBLE WITH THORACIC COMPRESSIONS**
WIEDERBELEBUNGSBEUTEL MIT MIT THORAXKOMPRESSIONEN KOMPATIBLER ABZWEIGLEITUNG
BALLON DE RÉANIMATION AVEC CONDUIT DE DÉRIVATION COMPATIBLE AVEC DES COMPRESSIONS THORACIQUES

(30) Priority: 27.06.2017 US 201762525421 P
(43) Date of publication of application: 06.05.2020
(73) Proprietor: Air Liquide Santé International, 75007 Paris (FR); Air Liquide Medical Systems, 92160 Antony (FR)
(72) Inventor: BOULANGER, Thierry, Media, PA 19063 (US); RICHARD, Jean-Christophe, 92160 Antony (FR); RIGOLLOT, Marceau, 92250 La Garenne Colombes (FR); GINER, Jean-Marc, 92250 La Garenne Colombes (FR)
(74) Representative: Air Liquide
(86) International application number: PCT/EP2017/080975
(87) International publication number: WO 2019/001752

(56) References cited:
- EP-A1- 1 512 426
- WO-A1-00/45883
- WO-A1-2009/047763
- US-A- 2 453 475
- US-A- 2 834 339
- US-A- 3 063 620
- US-A- 4 501 271
- US-A- 5 427 091
- US-A1- 2017 157 348

## Description

### Cross Reference to Related Application

This application claims the benefit of priority to U.S. Provisional Patent Application No. 62/525,421, filed June 27, 2017.

### Background

The present invention relates to an artificial respiration device, namely an artificial resuscitation bag that can be used for resuscitating a person, i.e. a patient, in state of cardiac arrest, and an installation comprising such an artificial resuscitation bag for resuscitating a person in state of cardiac arrest.

Cardiac arrest is a condition affecting hundreds of thousand people every year with a very poor prognosis.

One of the main lifesaving actions is to apply thoracic compressions or 'TCs' along with brief intervals of lung ventilation with a resuscitation bag. TCs are successive compressions and decompressions exerted on the thoracic cage of the person, i.e. the patient, in cardiac arrest. TCs aim at partially restoring inhalation and exhalation phases and therefore gas exchanges in the lungs, as well as promoting or restoring blood circulation toward the organs and especially the brain of the patient.

As these compressions and decompressions only mobilize small volumes of gas in and out of the patient's airways, it is advocated to perform regularly further gas insufflations to bring fresh O₂-containing gas into the lungs thereby enhancing the gas exchanges.

Usually, fresh O₂-containing gas is delivered by a resuscitation bag linked with an oxygen source and connected to the patient through a respiratory interface, typically a facial mask, a laryngeal mask, or an endotracheal tube.

To date, it is recommended to interpose 2 insufflations every 30 chest compressions, whereas the ideal rate of compressions, according to international guidelines, is between 100 and 120 compressions per minute (c/min).

However, several studies have shown that it is difficult for rescuers to correctly perform the resuscitation sequence and that the interruptions of TCs to initiate the insufflations with a resuscitation bag are often too long and deleterious with respect to the patient's outcome, as rapidly affecting the hemodynamic, i.e., in other words, offsetting the benefits of the TCs themselves.

A main goal of the present invention is to fix the problem encountered with current resuscitation bags, in particular to provide an improved resuscitation bag allowing continuous TCs and, when required, enabling insufflations of given volumes of fresh O₂-containing gas.

Artificial resuscitation bags are known from the prior art documents US 2834339 A, US 3063620 A, US 2017/0157348 A1, US 4501271 A, WO 2009/047763 A1 and US 5427091 A, of which the latter two disclose a structure with a deformable bag and a supplementary gas reservoir.

### Summary

A solution according to the present invention concerns an artificial resuscitation bag according to claim 1. The dependent claims define preferred embodiments of the invention. An artificial resuscitation bag according to the invention comprises:
- a deformable bag comprising a gas inlet and a gas outlet,
- a gas conduit in fluid communication with the gas outlet of the deformable bag, and
- a pneumatic valve comprising of an exhaust port cooperating with a membrane element for controlling the flow of gas exiting to the atmosphere through said exhaust port, said membrane element being arranged into an inner compartment of the pneumatic valve,
and further comprises:
- an overpressure valve arranged in the gas conduit,
- a first one-way valve arranged in the gas conduit between the overpressure valve and the pneumatic valve and
- a derivation conduct having:
   i) a first end fluidly connected to the gas conduit, between the gas outlet of the deformable bag and the overpressure valve, and
   ii) a second end fluidly connected to the inner compartment of the pneumatic valve,
   and further comprises:
   - a first conduit in fluid communication with the gas inlet of the deformable bag,
   - an oxygen line fluidly connected to said first conduit,
   - a one-way admission valve in fluid communication with the ambient atmosphere for allowing ambient air to enter into said first conduit and
   - a first exhaust valve arranged in the first conduit for venting excess of oxygen in the first conduit to the atmosphere,
   - a supplementary gas reservoir in fluid communication, via the first conduit element, with the gas inlet of the flexible bag, and
   - a second one-way valve arranged in the first conduit upstream of the deformable bag and configured to: prevent gas from the deformable bag from flowing backward in the first conduit and escaping via the first exhaust valve, and allow gas from the oxygen line, from the supplementary gas reservoir and from the one-way admission valve to flow via the first conduit into the deformable bag,
and wherein the pneumatic valve is arranged in the gas conduit, and further comprises a spring element acting on the membrane element for controlling the flow of gas exiting to the atmosphere through said exhaust port, said spring element being arranged into the inner compartment of the pneumatic valve.

Depending on the embodiment, an artificial resuscitation bag according to the present invention can comprise of one or several of the following additional features:
- the artificial resuscitation bag comprises a gas delivery conduit in fluid communication with the gas conduit for conveying at least part of the gas circulating into the gas conduit to a patient interface.
- the patient interface comprises of a respiratory mask or a tracheal cannula.
- the gas conduit conveys at least a part of the gas exiting the deformable bag through the gas outlet.
- the overpressure valve is configured to vent to the atmosphere at least part of the gas present in the gas conduit, when the gas pressure in the gas conduit exceeds a given threshold-value.
- the first one-way valve is configured for allowing a circulation of gas in the gas conduit only in the direction from the deformable bag toward the pneumatic valve.
- it further comprises an oxygen distribution system comprising a gas distributor and a by-pass line connected to said gas distributor.
- the gas distributor is arranged on the oxygen line.
- the by-pass line is fluidly connected to the gas distributor and to the patient interface.

Further, the present invention also concerns an installation for resuscitating a person in state of cardiac arrest comprising:
- an artificial resuscitation bag according to the present invention, and
- an O₂ source fluidly connected to the artificial resuscitation bag by means of an oxygen line, for providing oxygen to said artificial resuscitation bag.

### Brief Description of the Drawings

For a further understanding of the nature and objects for the present invention, reference should be made to the following detailed description, taken in conjunction with the accompanying drawings, in which like elements are given the same or analogous reference numbers and wherein:
- Figure 1 represents an embodiment of the resuscitation bag according to the prior art.
- Figure 2 illustrates an embodiment of the resuscitation bag according to the prior art.
- Figure 3A illustrates an embodiment of the resuscitation bag according to the prior art.
- Figure 3B illustrates an embodiment of the resuscitation bag according to the prior art.
- Figure 4 illustrates an embodiment of the resuscitation bag according to the prior art.
- Figure 5 illustrates an embodiment of the resuscitation bag according to the prior art.
- Figure 6 illustrates an embodiment of the resuscitation bag according to the prior art.
- Figure 7A illustrates an embodiment of the resuscitation bag according to the present invention.
- Figure 7B illustrates a control valve of a resuscitation bag according to an embodiment of the present invention.
- Figure 7C illustrates a control valve of a resuscitation bag according to an embodiment of the present invention.
- Figure 8 illustrates an embodiment of the resuscitation bag according to the present invention.
- Figure 9 illustrates an embodiment of the resuscitation bag according to the present invention.
- Figure 10 illustrates an embodiment of the resuscitation bag according to the present invention.
- Figure 11 illustrates an embodiment of the resuscitation bag according to the present invention.
- Figure 12 is another embodiment of the resuscitation bag, not according to the present invention.

### Description of Preferred Embodiments

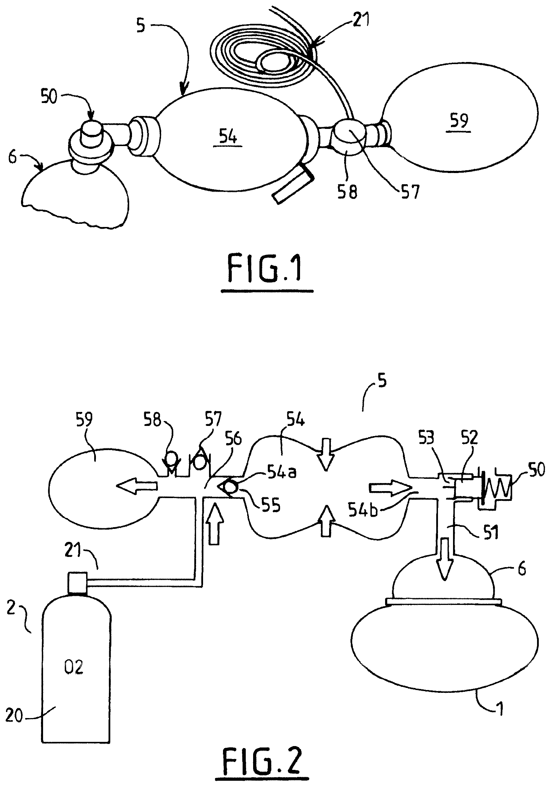

Figures 1 and 2 show a commercially available resuscitation bag **5** comprising of a respiratory interface **6** for feeding a respiratory gas to a patient, typically a respiratory mask, a flexible bag **54,** and a valve element **50,** such as a PEP valve, for diverting the gas in and out of the patient, during insufflation and exsufflation phases, and a source of an oxygen-containing gas **2,** such as or including a gas cylinder **20** containing oxygen, which is delivered during insufflation phases.

The flexible bag **54** is filled with fresh gas formed by a mixture of oxygen provided by an oxygen line **21** connected to the oxygen source **2** (cf. Fig. 2), typically a medical grade oxygen cylinder **20,** and ambient air provided by an admission valve **57** in fluid communication with the ambient atmosphere.

A supplementary gas reservoir **59** can be added to increase the availability of oxygen.

In Figure 2, a patient **1** is connected to the resuscitation bag **5,** via a respiratory interface **6,** e.g. a facial mask, a laryngeal mask or similar.

The oxygen source **2,** typically a cylinder **20** of medical grade oxygen, is fluidly connected via an oxygen line or tubing **21** and a first conduit element **56,** to the flexible bag **54,** the tubing **21** being fluidly connected to the first conduit element **56.** The first conduit element **56** is further fluidly communicating with the inlet orifice **54a** of the flexible bag **54.** The first conduit element **56** is arranged between the supplementary gas reservoir **59** and the flexible bag **54.**

Further, a first exhaust valve **58** is arranged in the first conduit element **56** for venting gas in the case of overpressure in the first conduit element **56.**

When the operator squeezes the flexible bag **54** to perform an insufflation of gas to the patient **1,** the flow of gas exiting the flexible bag **54** through its outlet orifice **54b** travels to the patient **1** into the lumen of a second conduit **51** that is fluidly connected to the respiratory interface **6,** such as a facial mask. At the same time, the flow of gas exiting the flexible bag **54** occludes the exhalation port **52** of a second exhaust valve **53** that is arranged in the second conduit **51,** i.e. downstream of gas bag **54,** as shown in Figure 2.

This generates positive pressure which, as a result, forces a second one-way valve **55** arranged upstream of the bag **54** to close thereby preventing the gas of bag **54** to flow backward, i.e. in the first conduit **56,** and to escape via the first exhaust valve **58.** Meanwhile, the flow of oxygen travelling in tubing **21** enters into the first conduit **56** element and fills the supplementary reservoir **59** that is fluidly connected to first conduit element **56.**

Due to the slightly positive pressure in first conduit element **56,** the air admission valve **57** is closed. In the case where the reservoir **59** becomes over-distended by the entering flow of gas, a pressure increase will occur in first conduit element **56** and the gas in excess will be vented to the ambient atmosphere by the first exhaust valve **58.** The opening pressure of the first exhaust valve **58** is close to 0, but slightly positive due to mechanical frictions.

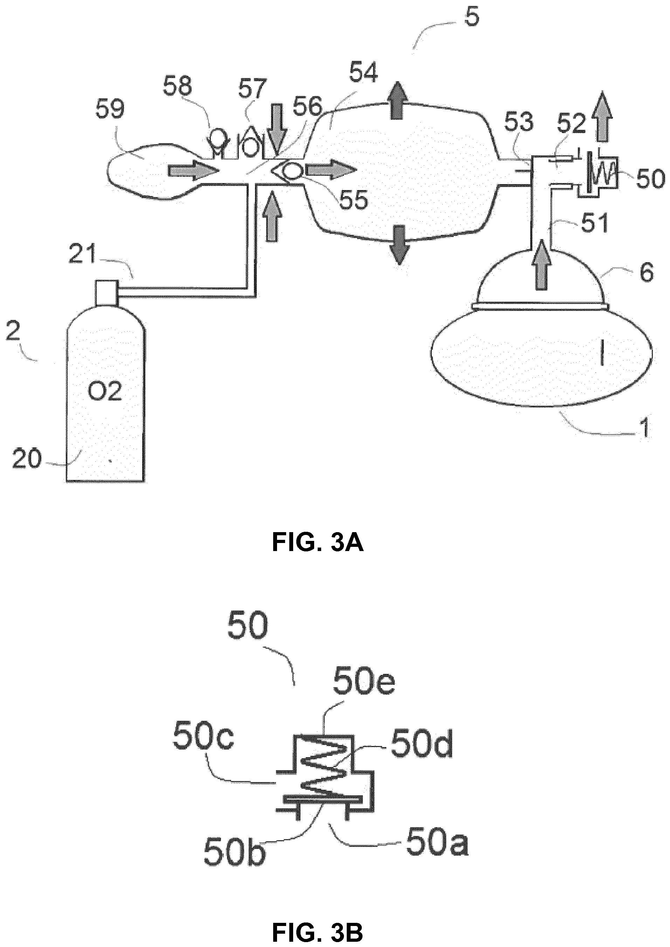

Figure 3A shows an expiration phase of the commercially available resuscitation bag **5** of Figures 1 and 2, when the operator has stopped squeezing the bag **54,** which bag **54** enters in an expansion phase due to a negative pressure that holds back the second exhaust valve **53** thereby opening the exhalation port **52.** The volume of gas accumulated in the patient's airways during the preceding inspiratory phase will travel through interface **6** and second conduit **51** before being vented to ambient atmosphere through the exhalation port **52.**

The resuscitation bag **5** includes a valve element **50** or PEP valve **50** (PEP = Positive Expiration Pressure) that creates a positive expiratory pressure, during exhalation phases, thereby helping keeping open the alveoli of the lungs of patient **1.**

As detailed in Figure 3B, such a PEP valve **50** typically comprises a spring **50d** arranged in a housing **50e,** which applies a constant force on a membrane **50b.** The gas pressure in the PEP valve inlet port **50a,** that is in fluid communication with exhalation port **52** and that applies on said membrane **50b,** has to be sufficiently high for exerting a force greater than the load of the spring **50d** for displacing the membrane **50b** backward and opening a fluidic pathway between the inlet port **50a** and an outlet port **50c** of the PEP valve inlet port **50a.** The fluidic pathway allows the gas pressure to escape through the outlet port **50c,** thereby allowing an expiration of gas by the patient **1.** It is possible to set the load of spring **50d** to different expiratory pressures, such as expiratory pressures corresponding to 5 cm H₂O, 10 cm H₂O, or 20 cm H₂O.

At the same time, the negative pressure generated in bag **54** will open the second one-way valve **55** that will: i) direct the gas flow from tubing **21** into bag **54** via conduit **56,** ii) empty reservoir **59** into bag **54** via conduit **56,** and iii) open the air admission valve **57** thereby allowing ambient air entering successively into conduit **56** and bag **54,** as shown in Figure 3A.

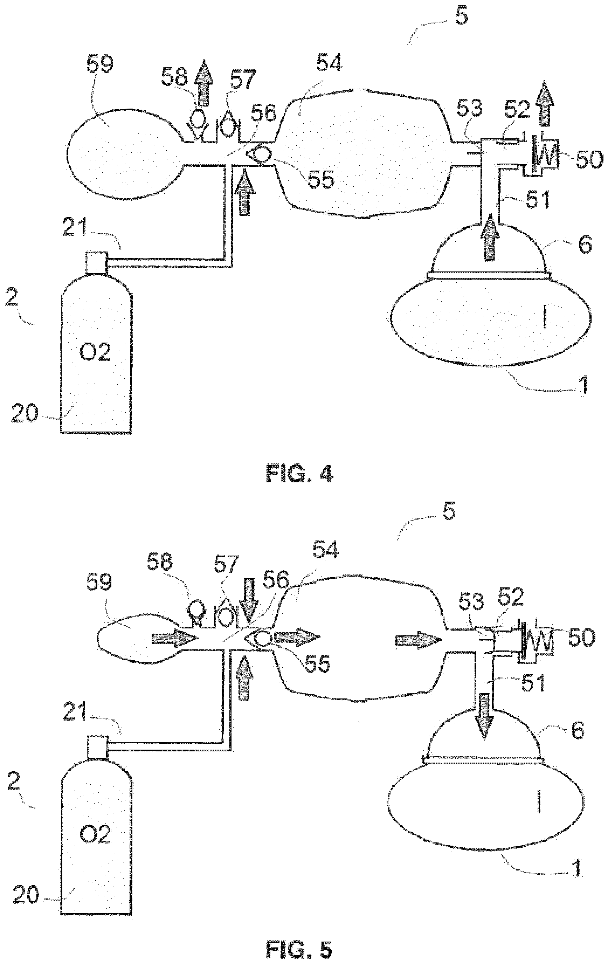

Further, Figures 4-6 show a sequence of thoracic compressions (TC) in association with the resuscitation bag **5** of Fig. 1, 2 and 3A.

In Figure 4, the resuscitation bag **5** is represented in its "rest" state, i.e. not active state, for example as it is before being used. The gas bag **54** and reservoir **59** are filled with gas and ready for an insufflation. The oxygen flowing from cylinder **20** and tubing **21** enters conduit **56** and is vented to the atmosphere through fist exhaust valve **58** acting as a safety valve.

When the bag **54** is in its "rest" state, the operator usually starts to exert thoracic compressions or TCs on the patient **1.** Due to the TCs, the second exhaust valve **53** is pushed back, i.e. closed, thereby occluding the fluidic pathway **52** between gas bag **54** and second conduit **51.** Indeed, a TC expels a small volume of gas from the patient's airways which travels backwardly through second conduit **51,** exhaust port **52,** and PEP valve **50.** Actually, PEP valve **50** creates a resistance force against expired gases, which will promote or restore blood circulation in the patient's body.

When a TC is relaxed, the patient **1** enters decompression and the airway pressure becomes negative as shown in Figure 5. The negative pressure closes PEP valve **50,** i.e. occlude the fluidic passage between ports **50a** and **50c** (cf. Fig. 3B), and air is delivered by bag **54,** thereby pushing the second exhaust valve **53** toward the exhaust port **52** for creating a fluidic passage between said gas bag **54** and second conduit **51.**

Meanwhile, the second one-way valve **55** allows: i) a first flow of gas, e.g. oxygen, to travel in tubing **21** and first conduit element **56,** and ii) a second flow of gas to exit reservoir **59** and to travel in first conduit element **56.**

Further, a third flow of gas, i.e. air, is allowed to penetrate into conduit **56** via the admission valve **57,** i.e. another one-way valve. These three flows of gas enter into bag **54** thereby filling said bag **54.**

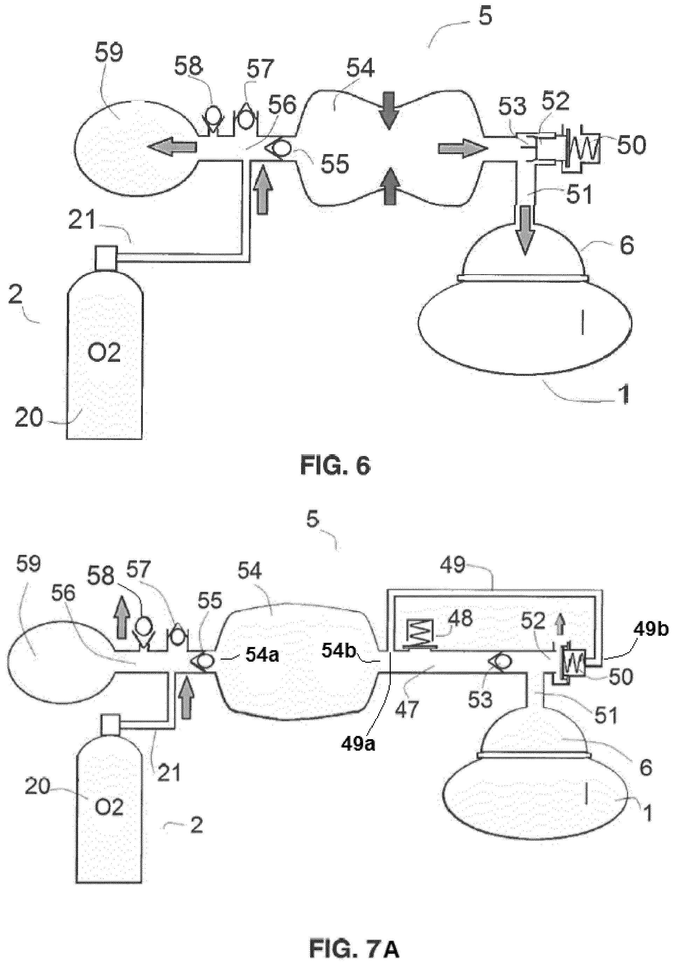

However, with such architecture a hazardous situation may exist as shown in Figure 6: when the operator performs an insufflation as described earlier, if a TC occurs during this insufflation phase, exhaust valve **53** and second one-way valve **55** will prevent any gas exhaust. This constitutes a risk for the patient **1** as an over-pressure will appear, which can be deleterious for the lungs of the patient **1.**

As shown in Figures 1-6, artificial resuscitation bags of the prior art do not allow to simultaneously performing safe and effective TCs and gas insufflations with the resuscitation bag. Indeed, with such known resuscitation bags, it is impossible to provide TCs during insufflations phases without risking overpressures in the lungs which will negatively impact outcomes for the patient. This is the reason why TCs must be interrupted when an insufflation is required.

The present invention proposes an artificial resuscitation bag **5** that can overcome the above issue.

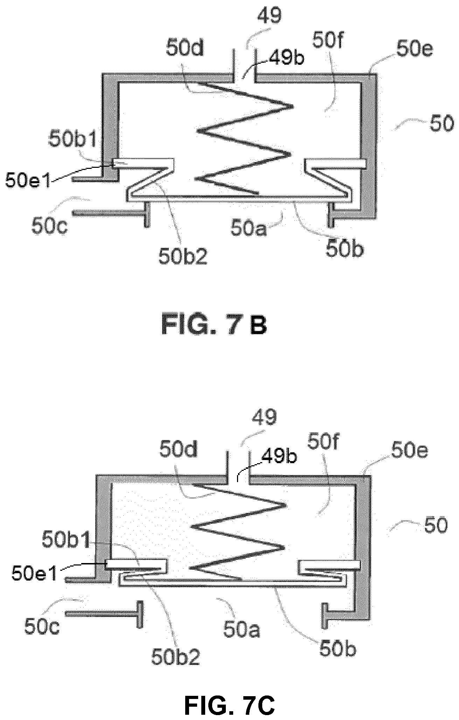

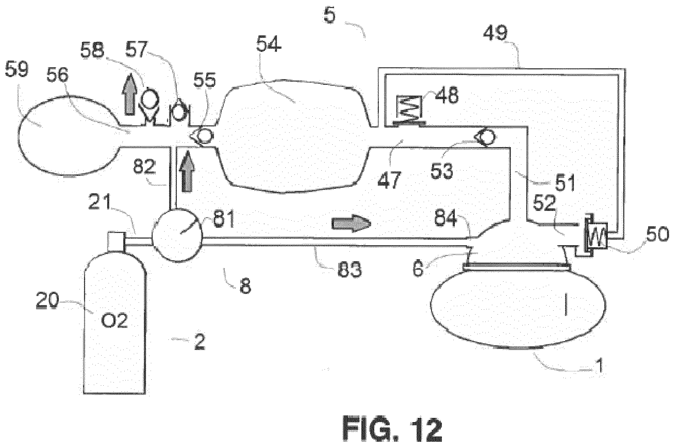

A first embodiment of an artificial resuscitation bag **5** according to the present invention is shown in Figures 7-11, whereas a second embodiment of an artificial resuscitation bag **5,** not according to the present invention, is shown in Figure 12.

Figure 7A shows a first embodiment of a resuscitation bag **5** according to the present invention, allowing TCs to be performed while insufflating gas.

In Figure 7A, the resuscitation bag **5** is in an initial state or "rest" state in case of a thoracic compression. The reservoir **59** is filled with oxygen, the oxygen being provided by the O₂ source **2,** namely the cylinder **20** delivering oxygen to reservoir **59** via tubing **21** and first conduit **56.** The first exhaust valve **58** is opened and vents the excess of oxygen to the atmosphere. The slight positive pressure in first conduit **56** keeps the one-way admission valve **57** closed. By mechanical transmission, this pressure will be equalized in all the parts behind the second one-way valve **55,** i.e. into bag **54** and subsequent components, such as conduits **47, 51** and **52.**

The control valve **50** of Figures 7B/7C works in differential mode. A deformable membrane **50b** is tightly attached by its lips **50b1** to grooves **50e1** in a rigid structure **50e,** which is the control valve **50** housing. A deformable portion **50b2** of membrane **50b** helps this membrane **50b** move forward or backward, depending on the conditions. At rest, this membrane **50b** prevents a fluidic connection between the inlet conduit **50a** and outlet conduit **50c,** as shown in Figure 7B. This is due to the force exerted by load spring **50d** on the membrane **50b,** as described previously. The force exerted by the load spring can be variable and set for example in a way that a pressure of 5 cm H₂O in inlet **50a** is necessary to move the membrane **50b** backward to perform a fluidic connection between inlet **50a** and outlet **50c,** as shown in Figure 7C.

However, the control valve **50** of Figures 7B/7C has a chamber **50f** which is fluidically connected to a derivation conduct **49** comprising a first end **49a** fluidly connected to the gas conduit **47,** between the gas outlet **54b** of the deformable bag **54** and the overpressure valve **48,** and a second end **49b** fluidly connected to the inner compartment **50f** of the pneumatic valve **50,** as shown in Figure 7A. Should the derivation conduct **49** provide a positive pressure, this pressure would add a force on top of the load spring **50d** which will in turn make it harder to open the fluidic connection between inlet **50a** and outlet **50c,** unless the pressure at inlet **50a** follows the increase of pressure in chamber **50f,** offsetting its effect.

As shown in Figure 7A, at the very onset of the TC, the pressure in conduits **47** and **51,** in derivation conduct **49** and consequently in chamber **50f** of the control valve **50** are equal. This means that only the load spring **50d** will oppose the rise of pressure resulting from the TC. Following the example set above, as soon as the pressure will exceed 5 cm H₂O in second conduit **51,** thus closing the first one-way valve **53,** also called second exhalation valve **53,** the control valve **50** will open to make a fluidic connection between inlet **50a** and outlet **50c,** allowing the volume expelled by the patient **1** to travel through interface **6,** conduits **51** and **52,** inlet **50a** and exhaust port, or outlet **50c.**

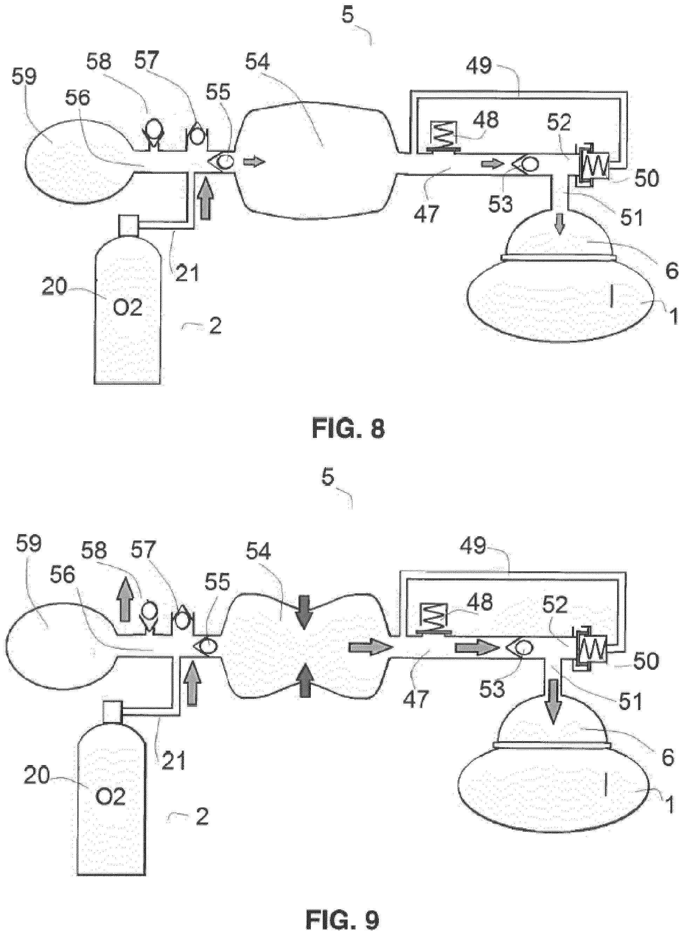

After the TC, follows a decompression phase as shown in Figure 8. The pressure in the patient's airways suddenly decreases to potentially subatmospheric pressures. As a consequence, the flow of oxygen in first conduit **56,** coming from tubing **21,** will be directed to the patient **1** to offset this decrease in pressure, opening the second one-way valve **55** and the second exhalation valve **53,** and traveling through bag **54** and conduits **47, 51** and interface **6.** In addition, the pressure across the control valve **50,** which is between derivation conduct **49** and therefore chamber **50f,** and conduit **52** and therefore inlet **50a** will be close to 0 and as a result the control valve **50** will be closed.

As a result, a direct fluidic pathway will be created between the oxygen supply in tubing **21** and patient **1.**

In Figure 9, the operator starts an insufflation by squeezing the bag **54** which will in turn open the first one-way valve **53.** By the same mechanism, the pressure across the control valve **50,** which is between derivation conduct **49** and therefore chamber **50f,** and conduit **52** and therefore inlet **50a** will be close to 0. As a result, the control valve **50** will remain closed, although the insufflation will create an increase in pressure in both sides of the control valve **50.** As a consequence, all the gas exiting the bag **54** will travel into conduits **47** and **51** and be delivered to the patient **1** via interface **6.**

On the other end of the bag, such positive pressure in the bag **54** will force the second one-way valve **55** to close and the oxygen coming from tubing **21** and entering conduit **56** will either fill the reservoir **59** or vent to the atmosphere through exhaust valve **58.**

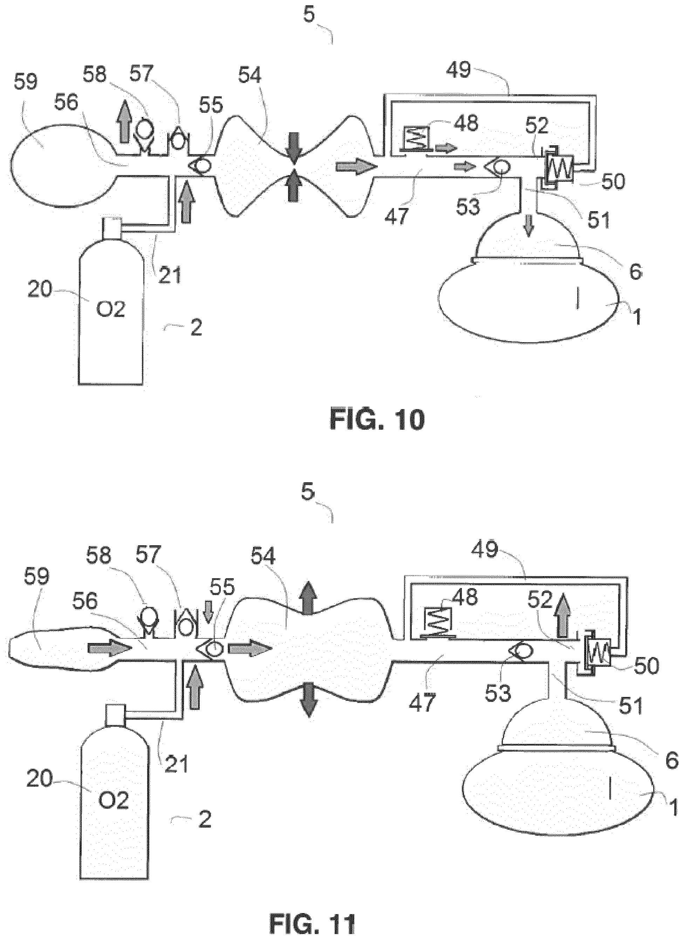

At some point during the insufflations, the pressure may become too high. The resuscitation bag of the present invention provides a means to control this pressure as shown in Figure 10. This function is made possible by PPEAK valve **48** which is of the same construction as the PEP valve **50** hereabove described (cf. Fig. 3B), although its load spring is set in a way that only a pressure greater than 20cm H₂O, for example, opens it and limits the pressure into conduits **47, 51** and patient's airways at this set value.

During the insufflation described with references to Figures 9 and 10, the control valve **50** assists the operator. Indeed, in case of a thoracic compression the pressure on the patient **1** side will increase, for instance above 20cm H2O if we consider the compression occurred while PPEAK valve **48** was limiting the pressure, and close the first one-way valve **53.** This will create an imbalance in terms of pressure between conduits **51, 52** and inlet **50a** and their counterpart, e.g. conduits **47,** derivation conduct **49** and chamber **50f.** As soon as this imbalance exceeds the spring load **50d,** causing a differential pressure of 5 cm H₂O, the control valve **50** will open and make a fluidic connection between inlet **50a** and outlet **50c,** allowing the volume expelled by the patient **1** to travel through interface **6,** conduits **51** and **52,** inlet **50a** and exhaust port, or outlet **50c.**

Figure 11 shows the expiration phase, when the operator has stopped squeezing the bag **54,** which enters an expansion phase. This creates a negative pressure which will open the second one-way valve **55,** which will in turn: i) direct flow from tubing **21** into bag **54** via the first conduit **56;** ii) empty reservoir **59** into bag **54** via first conduit **56;** and iii) open one-way admission valve **57** which will let ambient airflow into bag **54** via conduit **56.**

The same negative pressure will hold back the first one-way valve **53,** close PPEAK valve **48** and decrease the pressure in derivation conduct **49** which will in turn dramatically decrease the pressure in chamber **50f** of control valve **50.** As the pressure in the patient's airways is high as a consequence of the past insufflation, the control valve **50** opens to make a fluidic connection between inlet **50a** and outlet **50c,** allowing the volume expired by the patient **1** to travel through interface **6,** conduits **51** and **52,** inlet **50a** and exhaust port, or outlet **50c.** The control valve **50** will remain open until an equilibrium is met between pressure in conduits **51** and opening pressure of control valve **50,** defined by spring load **50d** which, by virtue of the description above, should be around. 5 cm H₂O. The patient **1** has returned to a low pressure level where subsequent thoracic compressions can occur, as described in Figure 7A.

The resuscitation bag **5** of the present invention has the ability to allow safe insufflations by limiting the pressure at the patient's airways while authorizing compression phases, therefore optimizing hemodynamic of the patient.

The resuscitation bag **5** of Figures 7-11 constitutes a great improvement over those of the prior art.

A second embodiment of the resuscitation bag **5,** not according to the present invention, that further enhances TCs, is shown in Figure 12.

Following a TC, as shown in Figure 7, the gas flowing into the patient **1,** during the thoracic decompression phase as illustrated in Figure 8, will partly be composed of the gas expelled from the patient **1** during TC and present in the interface **6** and conduits **51** and **52.**

This gas contains a "high" level of CO₂, which replaces valuable oxygen and further prevents the CO₂ clearance from the lung.

In many cases, it is advantageous:
- to lower as much as possible the space in which the CO₂ can be present, e.g. interface **6** and conduits **51** and **52,** and
- to "flush" out a maximum of CO₂ rich-gases, over the course of the resuscitation process.

In this aim, according to the second embodiment shown in Figure 12, the control valve **50** is arranged directly in the region of the interface **6** so as to be fluidly connected to interface **6** via conduit **52.** Thus, control valve **50** can more efficiently vent CO₂-enriched gases exhaled by patient **1** to the atmosphere, thereby avoiding CO₂ build-up into conduit **51.** Further, an oxygen distribution system **8** comprising a by-pass line **83** and a gas distributor **81** is provided. The by-pass line **83** is arranged between the gas distributor **81** fed by the oxygen source **2** and the interface **6.**

The inlet of the gas distributor **81** is fluidly connected to the oxygen source **2** via oxygen line or tubing **21.** In other words, the gas distributor **81** is arranged on the oxygen line **21** as shown in Figure 12.

The distributor **81,** when manually operated by the operator, diverts a portion of the total incoming oxygen flow either to the downstream portion **82** of the oxygen line **21** that is connected to resuscitation bag **5** via the first conduit **56,** or to the by-pass line **83** that is fluidly connected to the interface **6** via an admission port **84.**

By acting on gas distributor **81,** e.g. a proportional diverting valve, the operator can select/allocate the respective amounts of oxygen flowing into by-pass tubing **83** and further into the downstream portion **82** of the oxygen line **21.** The first oxygen flow conveyed by the downstream portion **82** of the oxygen line **21** enters into the first conduit **56** and, as already explained (cf. Fig. 7 and 8), when no gas insufflation is performed, helps keep a minimal positive pressure in the bag **54** and subsequent conduit **47,** derivation conduct **49** and chamber **50f,** thanks to exhaust valve **58.**

Further, the second oxygen flow conveyed by the by-pass tubing **83** enters into interface **6,** such as a respiratory mask, via the admission port **84.** As the oxygen flow is continuous, a pressure build-up occurs in interface **6** and conduit **52** and further a pressure imbalance across control valve **50** makes the fluidic connection between inlet conduit **50a** and outlet conduit **50c** to vent to the atmosphere, excessive flow, as hereinabove described in connection with Figure 7 to 11. Such a gas venting will also drag to the atmosphere any residual CO₂ from interface **6** and conduit **52.** Vented CO₂ is substituted by fresh oxygen delivered by by-pass line **83.**

Actually, an improved resuscitation bag according to the present invention brings the benefits to limit the level of CO₂ during the resuscitation process and promote oxygenation of the lungs.

## Claims

1. An artificial resuscitation bag (5) comprising:
- a deformable bag (54) comprising a gas inlet (54a) and a gas outlet (54b),
- a gas conduit (47) in fluid communication with the gas outlet (54b) of the deformable bag (54), and
- a pneumatic valve (50) comprising an exhaust port (50c) cooperating with a membrane element (50b) for controlling the flow of gas exiting to the atmosphere through said exhaust port (50c), said membrane element (50b) being arranged into an inner compartment (50f) of the pneumatic valve (50),
and further comprising:
- an overpressure valve (48) arranged in the gas conduit (47),
- a first one-way valve (53) arranged in the gas conduit (47) between the overpressure valve (48) and the pneumatic valve (50) and
- a derivation conduct (49) having:
i) a first end (49a) fluidly connected to the gas conduit (47), between the gas outlet (54b) of the deformable bag (54) and the overpressure valve (48), and
ii) a second end (49b) fluidly connected to the inner compartment (50f) of the pneumatic valve (50), and further comprising :
- a first conduit (56) in fluid communication with the gas inlet (54a) of the deformable bag (54),
- an oxygen line (21) fluidly connected to said first conduit (56), one-way admission valve (57) in fluid communication with the ambient atmosphere for allowing ambient air to enter into said first conduit (56) and
- a first exhaust valve (58) arranged in the first conduit (56) for venting excess of oxygen in the first conduit (56) to the atmosphere,
- a supplementary gas reservoir (59) in fluid communication, via the first conduit element (56), with the gas inlet (54a) of the flexible bag (54), and
- a second one-way valve (55) arranged in the first conduit (56) upstream of the deformable bag (54) and configured to: prevent gas from the deformable bag from flowing backward in the first conduit (56) and escaping via the first exhaust valve (58), and allow gas from the oxygen line (21), from the supplementary gas reservoir (59) and from the one-way admission valve (57) to flow via the first conduit (56) into the deformable bag (54),
and wherein the pneumatic valve (50) is arranged in the gas conduit (47), and further comprises a spring element (50d) acting on the membrane element (50b) for controlling the flow of gas exiting to the atmosphere through said exhaust port (50c), said spring element (50d) being arranged into the inner compartment (50f) of the pneumatic valve (50).

2. The artificial resuscitation bag according to claim 1, further comprising a gas delivery conduit (51) in fluid communication with the gas conduit (47) for conveying at least part of the gas circulating into the gas conduit (47) to a patient interface (6).

3. The artificial resuscitation bag according to claim 1, wherein the gas conduit (47) conveys at least a part of the gas exiting the deformable bag (54) through the gas outlet (54b).

4. The artificial resuscitation bag according to claim 1, wherein the overpressure valve (48) is configured to vent to the atmosphere at least part of the gas present in the gas conduit (47), when the gas pressure in the gas conduit (47) exceeds a given threshold-value.

5. The artificial resuscitation bag according to claim 1, wherein the first one-way valve (53) is configured for allowing a circulation of gas in the gas conduit (47) only in the direction from the deformable bag (54) toward the pneumatic valve (50).

6. The artificial resuscitation bag according to claim 1, wherein it further comprises an oxygen distribution system (8) comprising a gas distributor (81) and a by-pass line (83) connected to said gas distributor (81).

7. The artificial resuscitation bag according to claim 6, wherein the gas distributor (81) is arranged on the oxygen line (21).

8. The artificial resuscitation bag according to claims 2 and 6, wherein the by-pass line (83) is fluidly connected to the gas distributor (81) and to the patient interface (6).

9. An installation for resuscitating a person in state of cardiac arrest comprising:
- an artificial resuscitation bag (5) according to claim 1, and
- an O₂ source (2) fluidly connected to the artificial resuscitation bag (5) by means of the oxygen line (21), for providing oxygen to said artificial resuscitation bag (5).

## Patentansprüche

1. Beutel für künstliche Reanimation (5), umfassend:
- einen verformbaren Beutel (54), der einen Gaseinlass (54a) und einen Gasauslass (54b) umfasst,
- eine Gasleitung (47) in Fluidverbindung mit dem Gasauslass (54b) des verformbaren Beutels (54) und
- ein pneumatisches Ventil (50), das eine Auslassöffnung (50c) umfasst, die mit einem Membranelement (50b) zusammenwirkt, zum Steuern des Flusses von Gas, das durch die Auslassöffnung (50c) an die Atmosphäre austritt, wobei das Membranelement (50b) in einem Innenraum (50f) des pneumatischen Ventils (50) angeordnet ist,
und ferner umfassend:
- ein Überdruckventil (48), das in der Gasleitung (47) angeordnet ist,
- ein erstes Einwegventil (53), das in der Gasleitung (47) zwischen dem Überdruckventil (48) und dem pneumatischen Ventil (50) angeordnet ist, und
- eine Verzweigungsleitung (49), die aufweist:
i) ein erstes Ende (49a) in Fluidverbindung mit der Gasleitung (47) zwischen dem Gasauslass (54b) des verformbaren Beutels (54) und dem Überdruckventil (48) und
ii) ein zweites Ende (49b) in Fluidverbindung mit dem Innenraum (50f) des pneumatischen Ventils (50),
und ferner umfassend:
- eine erste Leitung (56) in Fluidverbindung mit dem Gaseinlass (54a) des verformbaren Beutels (54),
- eine Sauerstoffleitung (21) in Fluidverbindung mit der ersten Leitung (56),
- ein Einweg-Einlassventil (57) in Fluidverbindung mit der Umgebungsatmosphäre, um Eintreten von Umgebungsluft in die erste Leitung (56) zu erlauben, und
- ein erstes Auslassventil (58), das in der ersten Leitung (56) angeordnet ist, zum Ablassen von überschüssigem Sauerstoff in der ersten Leitung (56) an die Atmosphäre,
- ein zusätzliches Gasreservoir (59), über das erste Leitungselement (56) in Fluidverbindung mit dem Gaseinlass (54a) des flexiblen Beutels (54) und
- ein zweites Einwegventil (55), das stromaufwärts bezogen auf den verformbaren Beutel (54) in der ersten Leitung (56) angeordnet ist und gestaltet ist zum: Verhindern, dass Gas aus dem verformbaren Beutel in der ersten Leitung (56) rückwärts fließt und über das erste Auslassventil (58) entweicht, und Erlauben, dass Gas aus der Sauerstoffleitung (21), aus dem zusätzlichen Gasreservoir (59) und aus dem Einweg-Einlassventil (57) über die erste Leitung (56) in den verformbaren Beutel (54) fließt,
und wobei das pneumatische Ventil (50) in der Gasleitung (47) angeordnet ist und ferner ein Federelement (50d) umfasst, das auf das Membranelement (50b) einwirkt, zum Steuern des Flusses von Gas, das durch die Auslassöffnung (50c) an die Atmosphäre austritt, wobei das Federelement (50d) in dem Innenraum (50f) des pneumatischen Ventils (50) angeordnet ist.

2. Beutel für künstliche Reanimation gemäß Anspruch 1, ferner umfassend eine Gaszuführleitung (51) in Fluidverbindung mit der Gasleitung (47) zum Befördern wenigstens eines Teils des in die Gasleitung (47) zirkulierenden Gases zu einer Patientenschnittstelle (6).

3. Beutel für künstliche Reanimation gemäß Anspruch 1, wobei die Gasleitung (47) wenigstens einen Teil des Gases, das aus dem verformbaren Beutel (54) austritt, durch den Gasauslass (54b) befördert.

4. Beutel für künstliche Reanimation gemäß Anspruch 1, wobei das Überdruckventil (48) dafür gestaltet ist, wenigstens einen Teil des in der Gasleitung (47) vorhandenen Gases an die Atmosphäre abzulassen, wenn der Gasdruck in der Gasleitung (47) einen gegebenen Schwellenwert übersteigt.

5. Beutel für künstliche Reanimation gemäß Anspruch 1, wobei das erste Einwegventil (53) dafür gestaltet ist, Zirkulation von Gas in der Gasleitung (47) nur in der Richtung von dem verformbaren Beutel (54) zu dem pneumatischen Ventil (50) zu erlauben.

6. Beutel für künstliche Reanimation gemäß Anspruch 1, ferner umfassend ein Sauerstoffverteilungssystem (8), das einen Gasverteiler (81) und eine mit dem Gasverteiler (81) verbundene Bypassleitung (83) umfasst.

7. Beutel für künstliche Reanimation gemäß Anspruch 6, wobei der Gasverteiler (81) an der Sauerstoffleitung (21) angeordnet ist.

8. Beutel für künstliche Reanimation gemäß Ansprüchen 2 und 6, wobei die Bypassleitung (83) in Fluidverbindung mit dem Gasverteiler (81) und der Patientenschnittstelle (6) steht.

9. Einrichtung für die Reanimation einer Person in einem Zustand von Herzstillstand, umfassend:
- einen Beutel für künstliche Reanimation (5) gemäß Anspruch 1 und
- eine O₂-Quelle (2), die über die Sauerstoffleitung (21) in Fluidverbindung mit dem Beutel für künstliche Reanimation (5) steht, zur Bereitstellung von Sauerstoff an den Beutel für künstliche Reanimation (5).

## Revendications

1. Ballon de réanimation artificielle (5) comprenant :
- un ballon déformable (54) comprenant une entrée de gaz (54a) et une sortie de gaz (54b),
- un conduit de gaz (47) en communication fluidique avec la sortie de gaz (54b) du ballon déformable (54), et
- une vanne pneumatique (50) comprenant un orifice d'échappement (50c) coopérant avec un élément à membrane (50b) pour réguler le flux de gaz sortant vers l'atmosphère par ledit orifice d'échappement (50c), ledit élément à membrane (50b) étant agencé dans un compartiment interne (50f) de la vanne pneumatique (50), et comprenant en outre
- une vanne de surpression (48) agencée dans le conduit de gaz (47),
- une première vanne unidirectionnelle (53) agencée dans le conduit de gaz (47) entre la vanne de surpression (48) et la vanne pneumatique (50) et
- un conduit de dérivation (49) comportant :
i) une première extrémité (49a) reliée fluidiquement au conduit de gaz (47), entre la sortie de gaz (54b) du ballon déformable (54) et la vanne de surpression (48), et
ii) une seconde extrémité (49b) reliée fluidiquement au compartiment interne (50f) de la vanne pneumatique (50),
et comprenant en outre
- un premier conduit (56) en communication fluidique avec l'entrée de gaz (54a) du ballon déformable (54),
- une ligne d'oxygène (21) reliée fluidiquement audit premier conduit (56),
- une vanne d'admission unidirectionnelle (57) en communication fluidique avec l'atmosphère ambiante pour permettre que l'air ambiant entre dans ledit premier conduit (56) et
- une première vanne d'échappement (58) agencée dans le premier conduit (56) pour évacuer l'excès d'oxygène dans le premier conduit (56) vers l'atmosphère,
- un réservoir de gaz supplémentaire (59) en communication fluidique, par le biais du premier élément de conduit (56), avec l'entrée de gaz (54a) du ballon souple (54), et
- une seconde vanne unidirectionnelle (55) agencée dans le premier conduit (56) en amont du ballon déformable (54) et conçue pour : empêcher le flux de gaz provenant du ballon déformable de retourner dans le premier conduit (56) et de s'échapper par le biais de la première vanne d'échappement (58), et permettre que le gaz provenant de la ligne d'oxygène (21), du réservoir de gaz supplémentaire (59) et de la vanne d'admission unidirectionnelle (57) s'écoule par le biais du premier conduit (56) dans le ballon déformable (54),
et dans lequel la vanne pneumatique (50) est agencée dans le conduit de gaz (47), et comprend en outre un élément de ressort (50d) agissant sur l'élément à membrane (50b) pour réguler le flux de gaz sortant vers l'atmosphère à travers ledit orifice d'échappement (50c), ledit élément de ressort (50d) étant agencé dans le compartiment interne (50f) de la vanne pneumatique (50).

2. Ballon de réanimation artificielle selon la revendication 1, comprenant en outre un conduit de distribution de gaz (51) en communication fluidique avec le conduit de gaz (47) pour acheminer au moins une partie du gaz circulant dans le conduit de gaz (47) vers une interface patient (6).

3. Ballon de réanimation artificielle selon la revendication 1, dans lequel le conduit de gaz (47) achemine au moins une partie du gaz sortant du ballon déformable (54) à travers la sortie de gaz (54b).

4. Ballon de réanimation artificielle selon la revendication 1, dans lequel la vanne de surpression (48) est conçue pour évacuer vers l'atmosphère au moins une partie du gaz présent dans le conduit de gaz (47), lorsque la pression gazeuse dans le conduit de gaz (47) dépasse une valeur seuil donnée.

5. Ballon de réanimation artificielle selon la revendication 1, dans lequel la première vanne unidirectionnelle (53) est conçue pour permettre une circulation du gaz dans le conduit de gaz (47) uniquement dans la direction allant du ballon déformable (54) à la vanne pneumatique (50).

6. Ballon de réanimation artificielle selon la revendication 1, comprenant en outre un système de distribution d'oxygène (8) comprenant un distributeur de gaz (81) et une ligne de dérivation (83) reliée audit distributeur de gaz (81).

7. Ballon de réanimation artificielle selon la revendication 6, dans lequel le distributeur de gaz (81) est agencé sur la ligne d'oxygène (21).

8. Ballon de réanimation artificielle selon les revendications 2 et 6, dans lequel la ligne de dérivation (83) est reliée fluidiquement au distributeur de gaz (81) et à l'interface patient (6).

9. Installation pour la réanimation d'une personne en état d'arrêt cardiaque comprenant :
- un ballon de réanimation artificielle (5) selon la revendication 1, et
- une source d'O₂ (2) reliée fluidiquement au ballon de réanimation artificielle (5) au moyen de la ligne d'oxygène (21), pour fournir de l'oxygène audit ballon de réanimation artificielle (5).
